# EUROPEAN PATENT SPECIFICATION

(11) **EP 1 942 754 B1**
(45) Date of publication and mention of the grant of the patent: **08.12.2010**
(21) Application number: 06831827.8
(22) Date of filing: 02.10.2006
(51) Int. Cl.: A24F 47/00

(54) **ELECTRICAL SMOKING SYSTEM**
ELEKTRISCHES RAUCHSYSTEM
SYSTEME ELECTRIQUE POUR FUMEUR

(30) Priority: 30.09.2005 US 722035 P; 30.09.2005 US 722036 P
(43) Date of publication of application: 16.07.2008
(73) Proprietor: Philip Morris Products S.A., 2000 Neuchâtel (CH)
(72) Inventor: ADAMS, John, M., Mechanicsville, VA 23116 (US); CROWE, William, J., Chester, VA 23831 (US); HEARN, John, R., Midlothian, VA 23112 (US); LEE, Robert, E., Richmond, VA 23238 (US); STEVENSON, Brett, W., Richmond, VA 23234 (US); YANG, Zuyin, Midlothian, VA 23114 (US); BAGGETT, James, D., Jr., Richmond, VA 23225-7419 (US); HAIRFIELD, John, R., Jr., Chester, VA 23836 (US); LARSON, Steven, J., Richmond, VA 23535 (US); RIPLEY, Robert, L., Midlothian, VA 23113 (US); WRENN, Susan, E., Chesterfield, VA 23832 (US)
(74) Representative: Marlow, Nicholas Simon
(86) International application number: PCT/IB2006/003842
(87) International publication number: WO 2007/042941

(56) References cited:
- EP-A1- 0 503 767
- EP-A2- 0 430 559
- WO-A-2007/039794
- DE-A1- 19 854 005
- US-A- 6 095 153

## Description

This disclosure relates generally to tobacco smoking systems using electrical energy rather than combustion, see e.g. EP-A-0 430 559. More particularly, the smoking system disclosed here generates an aerosol through conductive and/or convective combustionless heating of tobacco by an electrical heating source.

### SUMMARY

A quantity of tobacco is placed in contact with a heating system. Passageways are provided for air to move through the heating system and the tobacco. The heating system raises the temperature of the tobacco to the range of about 150°C to about 220°C by direct contact with the tobacco, by convective heat transfer to the tobacco, and/or by heating the air which in turn heats the tobacco. The heated tobacco releases volatiles which subsequently cool to form an aerosol for delivery from the heating system.

The tobacco may have a variety of shapes including without limitation a pillow shape, a generally rotationally symmetric shape, a generally cylindrical plug, a generally cylindrical shell, a generally circular disk, a plug shape, a pellet shape, a cigarette shape, and the like.

The heating system may also have a variety of configurations. By way of example, and without limitation, the heating system may include a heating element such as a generally cylindrical heated shell with both ends open, a generally cylindrical heated shell with a closed end, an insertable heating element, a heated disk, a pair of heated disks, or the like. Such heating elements may be fabricated from an electrically resistive material which heats when electrical current passes through it. Such heating elements may also include either internal or external heating devices such as wires. Air may pass axially through the tobacco and the heating system. Alternatively air may enter the tobacco radially and exit substantially axially. In addition, the heating system may be arranged such that air is preliminarily heated before being directed into the tobacco.

If desired, a mouthpiece, with or without a filter, may be used with the heating system both to define a cooling region for the tobacco volatiles, and to direct the resulting aerosol to the consumer.

### BRIEF DESCRIPTION OF THE DRAWINGS

The accompanying drawings depict several embodiments of the electrically heated tobacco smoking system of this disclosure. In the accompanying drawings, like reference numerals are applied to like elements.
FIG. 1 is a cross-sectional view of an electrically heated tobacco smoking system.
FIG. 2 is a cross-sectional view of the electrically heated tobacco smoking system taken along the line 2-2 of FIG. 1.
FIG. 3 is a cross-sectional view of another embodiment of the electrically heated tobacco smoking system.
FIG. 4 is a cross-sectional view taken along the line 4-4 of FIG. 3.
FIG. 5 is a cross-sectional view of the electrically heated smoking system of FIG. 1 including a mouthpiece.
FIG. 6 is an end view of the electrically heated tobacco smoking system of FIG. 5.
FIG. 7 is a cross-sectional view of a third embodiment of the electrically heated tobacco smoking system.
FIG. 8 is an end view of the electrically heated tobacco smoking system of FIG. 7.
FIG. 9 is a cross-sectional view of an electrically heated tobacco smoking system using a pillow of tobacco material.
FIG. 10 is an end view of the electrically heated tobacco smoking system of FIG. 9.
FIG. 11 is a cross-sectional view of an electrically heated tobacco smoking system having radial inflow of air.
FIG. 12 is an end view of the electrically heated tobacco smoking system of FIG. 11.
FIG. 13 is a cross-sectional view of an electrically heated tobacco smoking system having counter-current heat exchange.
FIG. 14 is an end view of the electrically heated tobacco smoking system of FIG. 13.
FIG. 15 is a cross-sectional view of an electrically heated tobacco smoking system with a toroidal tobacco mass.
FIG. 16 is a cross-sectional view taken along line 16-16 of FIG. 15.

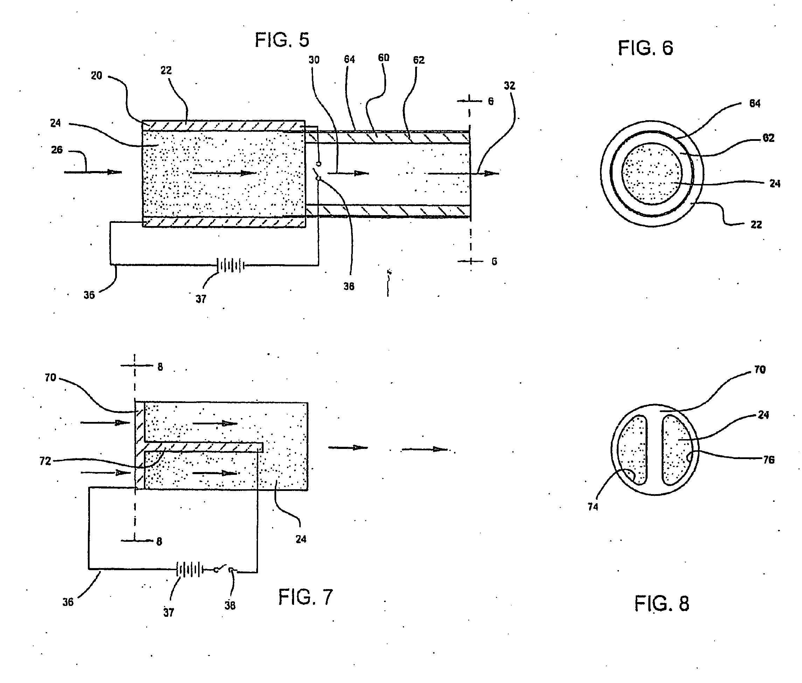

FIG. 7 and FIG. 8 relate to embodiments of the present invention. The other figures relate to alternative embodiments.

### DETAILED DESCRIPTION

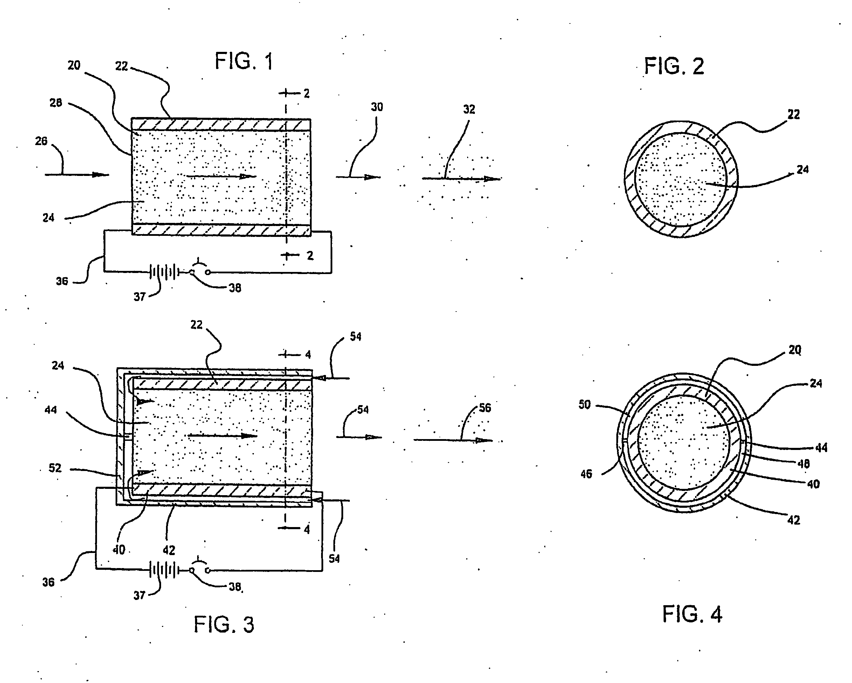

Turning to FIG. 1, an electrically operated tobacco smoking system 20 according to this disclosure is depicted. The smoking system 20 includes at least a heating system 22 and a tobacco mass that may comprise a plug 24 of cut filler tobacco. The heating system may be an inductively heated arrangement, a resistively heated arrangement, a radiantly heated arrangement, or a convectively heated arrangement. Here, resistively heated arrangements will be described as currently preferred embodiments. The tobacco plug 24 preferably weighs in the range of 5mg to 500mg. For single and multiple puff applications, weights in the range of 5mg to 150mg, are more preferable. Weights in the range of 300mg to 500mg, are more preferable where tobacco is to be used on multiple occasions. The tobacco mass may be made from any type of tobacco, or from any portion of the tobacco plant including, without limitation, sheet products, dust, leaf, stem, and combinations thereof. In the final form, the tobacco mass could involve any type of common tobacco processing steps including, without limitation, blending, flavoring, and the like.

If desired, the tobacco plug 24 may include a paper or mesh cover or carrier to facilitate handling. When paper is used around the tobacco plug, the paper substrate may have porosity and weight selected according to the particular shape of the heating chamber and the manner in which the tobacco plug 24 is to be used. For example, where the tobacco plug 24 is replaceable, the paper needs to have sufficient strength to maintain integrity of the tobacco plug during handling, removal, and insertion. Moreover, where the paper covers the principal movement of air through the smoking system, the paper needs to have sufficient porosity to allow air movement therethrough. Where a mesh material is selected, mesh opening should be sized small enough to contain a substantial majority, if not all, of the cut filler tobacco particles.

The tobacco mass may also be formed from tobacco particles. Whether particles or other tobacco cuts are used, the tobacco itself may function as a binder to hold tobacco pieces in a preferred shape. If desired, the tobacco used in the system can be pretreated to enhance flavor generation. The tobacco mass may be rotationally symmetric in shape or configuration. Moreover, the tobacco mass may be generally cylindrical, disk-like, or generally toroidal. Where a cylindrical configuration is adopted, it may be solid or shell-like with an open center. The particular shape of the tobacco mass is preferably adapted to the shape of the heating apparatus.

The heating system 22 substantially encloses or surrounds a substantial portion of the tobacco mass such that at least part of the surface of the tobacco mass conforms to the heating apparatus. An actuation system connected with the heating system 22 is operable to electrically energize the heating system 22. The actuation system may include a source of electrical energy such as one or more batteries 37. To preserve battery life and to control activation of the heating system 22, a suitable switch 38 may be connected in series with the batteries 37. Depending on the application, the switch 38 may be a push-button switch, a flow sensing device, or a puff sensing device. A suitable conductor 36 connects the batteries 37, the switch 38, and the heating system 22 in series.

The heating system 22 is operable to heat the tobacco mass to a temperature in the range of about 150°C to about 220°C to release flavorful volatiles without reaching the tobacco kindling temperature and without generating smoke and/or ash. Further, the heating system defines a heat transfer channel or pathway through which air is directed.

As shown, the heating system 22 may comprise a generally cylindrical shell having both ends open. As best seen in FIG. 2, the generally cylindrical shell surrounds the sides of the tobacco plug 24. The heating system 22 is electrically energized to generate heat. To that end, the heating system 22 may be connected to a suitable source of electrical energy, such as, for example, domestic power grid, portable power generating devices like an automobile cigarette lighter, batteries, and any other suitable conventional source. The source of energy must be capable of delivering heat from the heating system 22 to the tobacco plug 24 so that the tobacco in the plug is raised to a temperature in the range of about 150°C to about 220°C. Within this temperature range, the tobacco releases flavorful volatiles that produce a satisfying experience while generating little to no visible smoke.

The heating system 22 may operate continuously for a period of time corresponding to the length of time normally used to smoke a cigarette. Conventionally, that length of time is typically taken as about 5 to 10 minutes. Alternatively, the heating system 22 may operate intermittently, on demand, for example in response to puffs by the system user.

In use, the heating system 22 is activated, for example, by a puff sensor or a suitable "on-off" device. Ambient air 26 is drawn through the open end 28 of the heating system 20 and into the tobacco plug 24. The heating system 22 closely conforms to the periphery of the tobacco plug 24 and heats the tobacco plug 24 to the desired temperature range (about 150°C to about 220°C) by conductive and/or convective heat transfer to release flavorful volatiles from the tobacco. As the ambient air 26 moves through the tobacco plug 24, the air 26 entrains the released tobacco volatiles and distills those volatiles by cooling them. As the air with entrained volatiles leaves the second end of the heating system 22, see arrow 30, exposure to ambient air further cools the entrained volatiles to form an aerosol 32 which is delivered from the heating system 22.

If desired, an aerosol former may be added to the tobacco plug 24. Suitable aerosol formers include, for example, glycerol, propylene glycol, triacetin, and the like, as well as mixtures thereof. Concentrations of aerosol former in the range of about 0 to about 25% by weight can be used. The aerosol formers also enhance tobacco involvement in the aerosol formation.

After the tobacco plug 24 has been used, it may be removed from the heating system. 22 and replaced with a fresh plug or cartridge. The spent tobacco plug 24 may be discarded.

It will be appreciated by those skilled in the art that the electric tobacco smoking system described herein provides numerous advantages. For example, a smaller amount of tobacco is used so tobacco supplies can be extended. By using less tobacco, the disposable waste from the smoking experience is also reduced. In addition, since the heating system releases principally the flavorful volatiles from the tobacco which may be perceived as more pleasing than the sidestream aroma resulting from cigarette combustion.

The tobacco plug 24 is not subject to actual combustion as occurs with lit-end smoking articles. Because the electric tobacco smoking system described here does not have a smoldering coal, it presents less risk of starting a fire when carelessly handled than a conventional lit-end cigarette. For the same reason, the smoking system herein described requires less cleaning as there is essentially no ash with which to contend. Furthermore, the disposable waste which does exist has less residual aroma due to the lack of combustion. Moreover, absence of smoke should obviate non-smoker objections to second-hand smoke.

Of course, various other embodiments of the electric tobacco smoking system are not only possible but also are within the scope of this disclosure. For example (see FIG. 3), the tobacco plug 24 may be substantially enclosed by the heating system 22. In this embodiment, the heating system may include a heater 40 that is received within a generally cylindrical shroud 42. The actuation system of FIG. 3 is like that of FIG. 1 and includes conductors 36, batteries 37, and a switch 38.

To provide one or more channels for air flow around the heater 40, the heater 40 and/or the shroud 42 may include two or more radially outwardly extending ribs 44, 46 (see FIG. 4) which are operable to space the heater 40 from the shroud 42. The ribs 44, 46 may extend longitudinally along the outer surface of the heater 40. The ribs 44, 46, cooperate with the shroud 42 and the heater 40 to define a pair of preheating chambers or channels 48, 50 through which air can enter the smoking system as indicated by the arrows in FIG. 3. At least one of the ribs 44, 46 also extends axially beyond the heater 40 and functions as a spacer to hold the heater 40 away from the end wall 52 of the shroud 42.

The cross-sectional area of the channels 48, 50 may be selected as desired. For example, it may be desired to provide a flow area around the heater 40 which is equivalent to the flow area through the inside of the heater 40. Or, it may be desired to provide a flow area equivalent to the flow area through the tobacco plug 24. Regardless of the flow area selected, the heater 40 is preferably spaced from the end wall 52 by a sufficient distance that the flow area at the inner end of the heater 40 is at least as large as the flow area through the channels 48, 50. In this manner, the puff resistance (resistance-to-draw) is defined by the tobacco plug rather than by the assembly of the heater 40 and the shroud 42.

With the arrangement of FIGs. 3 and 4, air entering the electric tobacco smoking system, see arrows 54, flows axially through the preheating chambers or channels 48, 50 between the heater 40 and the shroud 42. During that time, the air is heated by thermal energy that might otherwise escape from the outer surface of the heater 40. That air then passes around the end of the heater, and flows through the tobacco plug 24 where it picks up flavorful volatiles from the tobacco heated by the heater 40. As the air 54 leaves the heater 40, it cools so that the volatiles condense and form the aerosol 56 for delivery from the heating system 22.

If desired, the spacing between the heater 40 and the shroud 42 may be accomplished with structures other than the longitudinal ribs described above. For example, discontinuous ribs, radially extending pins, and baffles may be used as desired. Baffles might be used to improve heat transfer to air under some circumstances.

Thus, the embodiment of FIGs. 3 and 4 improves the heat transfer to air and is more efficient than embodiments without the shroud 42.

The electric tobacco smoking system of this disclosure may also include a mouthpiece 60 (see FIGs. 5 and 6) having an end in fluid communication with the tobacco mass. The mouthpiece 60 may be constructed and arranged such that it has a cross-sectional configuration corresponding to the cross section of the heater assembly 22. In addition, the mouthpiece 60 may include a generally cylindrical shell 62 and an attachment sheath 64. The shell 62 preferably extends outwardly from the tobacco plug 24, has an open interior, and provides a channel or passage to deliver the aerosol 32. The shell 62 also functions to provide structural support for the heater 22 and tobacco plug 24. The sheath 64 preferably surrounds the shell 62 and may be constructed from paper or any other suitable conventional material.

The sheath 64 extends beyond the end of the shell 62 and into an opening within the heating assembly 22, which opening may be between the heating assembly 22 and the tobacco mass. By attaching the sheath 64 to the electric tobacco smoking system 20, the combination of the smoking system 20 and the mouthpiece 60 cosmetically resembles a conventional cigarette.

When more than one mouthpiece 60 will be used with the smoking system 20, or when one mouthpiece 60 will be used with more than one smoking system 20, the sheath may be fabricated from a sufficiently rigid material that the mouthpiece can be removably attached to the smoking system 20 by inserting the extending portion of the sheath 64 between the heating assembly 22 and the tobacco plug 24. For some applications, it may also be desirable to provide a suitable filter (not shown) such as a plug of cellulose acetate in the discharge end of the mouthpiece 60. Typically, such a filter would be located at an end of the mouthpiece. The actuation system of FIG. 5 is like that of FIG. 1 and includes conductors 36, batteries 37, and a switch 38; however, in FIG. 5, the switch 38 may.be a puff sensing device or an air flow sensing device.

Another arrangement for the heating assembly is shown in FIGs. 7 and 8. In this embodiment, the heating assembly includes an end piece 70 and a projection 72. The projection 72 may, for example, be a rod constructed and arranged so that it can be inserted into and received by the tobacco plug 24. The axial length of the projection 72 is selected so that it does not extend all the way through the tobacco plug. The end piece or heater disk 70 includes air passages or openings 74, 76 through which air can be introduced into the tobacco plug 24. At least one of the end piece 70 and the projection 72 may be energized to generate heat, e.g., by resistance heating or induction heating. Presently it is contemplated that both the end piece 70 and the projection 72 are capable of generating heat. However, if the tobacco plug 24 has a small diameter compared to the transverse dimensions of the projection 72, sufficient heat may be generated by the projection 72 that additional heat from the end piece 70 is not needed. The actuation system of FIG. 7 is like that of FIG. 1 and includes conductors 36, batteries 37, and a switch 38.

While the end piece 70 is depicted with two openings, the number, arrangement, and shape of the openings can vary. For example, a pattern of circular holes could be used if desired. Where the end piece 70 is heated, heat transfer to the air passing through the openings can be increased by using smaller openings.

If desired, the heating assembly 70, 72 and the associated tobacco plug 24 may be enclosed in other structures. For example, a cylindrical shell may surround the tobacco plug, or the tobacco plug along with the end piece 70. Such a cylindrical shell could also include a mouthpiece portion.

The embodiments discussed above include a generally cylindrical plug of tobacco; however, that shape for the tobacco is not critical. The cross section may be circular, as shown. Alternatively, the cross section can be uniform or non-uniform and can have any desired shape such a polygonal, elliptical, oval, toroidal, and the like. Moreover, the tobacco can have other suitable shapes as will be apparent to those skilled in the art. For example, the tobacco can be shaped as a pillow, i.e., a unit shaving a depth which is small compared with the transverse dimension. The pillow may be circular, rectangular, polygonal, polygonal with rounded corners, toroidal, or the like.

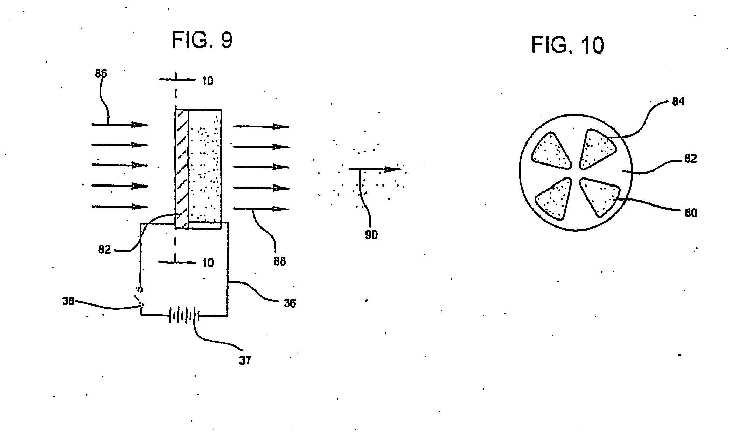

A generally circular embodiment of the pillow arrangement is depicted in FIGs. 9 and 10. Here, the pillow 80 is fashioned from cut filler tobacco. As desired, the pillow 80 may include a permeable membrane surrounding it to hold its shape and facilitate handling. The thickness of the pillow 80 may be in the range of 10-80% of a nominal transverse dimension of the pillow 80. In the context of FIG. 9, the nominal transverse dimension would be measured in the vertical direction and would correspond to the diameter of the pillow 80. The pillow thickness would be measured in the horizontal direction, axially in the direction of airflow.

The heating system 82 for this embodiment comprises a heating plate in heat transfer relationship with the tobacco pillow 80. The actuation system of FIG. 9 is like that of FIG. 1 and includes conductors 36, batteries 37, and a switch 38. The plate 82 may be a generally circular disk and may include a plurality of openings 84 to permit air to flow into the pillow 80. Those openings 84 may have any desired shape. In some applications, the openings 84 will be sized to provide heat transfer from the heating plate 82 to air (see arrows 86) before the air enters the tobacco pillow 80.

Thermal energy from the plate 82 is applied to the tobacco pillow 80 by conduction and convection so that the temperature of the pillow is raised to release the flavorful volatiles. As with other embodiments, that temperature lies in the range of about 150°C to about 220°C. As air leaves the tobacco pillow with the entrained flavorful volatiles (see arrows 88), the flavorful volatiles cool to form an aerosol (see arrow 90).

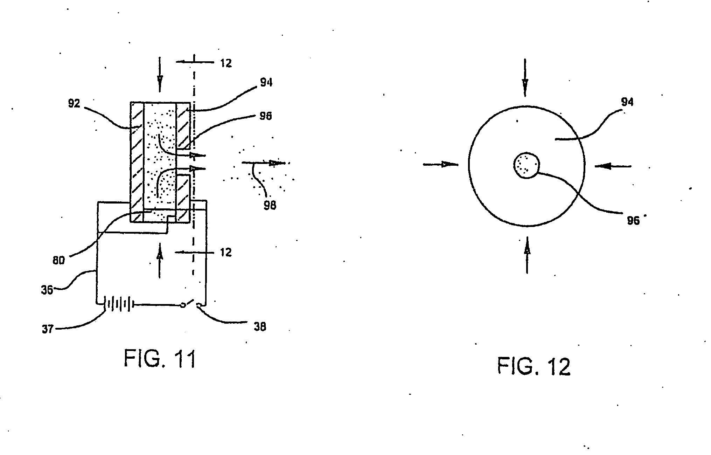

A pillow 80 having radial air inflow and axial outflow is shown in FIGs. 11 and 12. In this embodiment, the heating arrangement may include a disk-shaped heater 92 and an annular heater 94. The disk heater 92 and the annular heater 94 are spaced from one another by a distance corresponding to the thickness of the pillow 80 and sandwich the pillow therebetween. Both the disk heater 92 and the annular heater 94 have heat transfer relationship with the pillow 80 so that the heaters 92, 94 can raise the temperature of tobacco in the pillow 80 to a temperature sufficient to release the flavorful volatiles (i.e., about 150°C to about 220°C). The actuation system of FIG. 11 is like that of FIG. 1 and includes conductors 36, batteries 37, and a switch 38.

In this arrangement, ambient air enters the pillow 90 in a generally radial direction, for example, in response to inhalation. As the ambient air passes through the pillow 80, its temperature rises by heat transfer from one or both of the heaters 92, 94 and it entrains volatiles released from the tobacco. Air with the flavorful tobacco volatiles, turns axially and leaves the assembly through a generally circular orifice or opening 96 in the center of the annular heater 94. The air with entrained tobacco volatiles cools as it leaves the heater and condenses to form an aerosol (arrow 98).

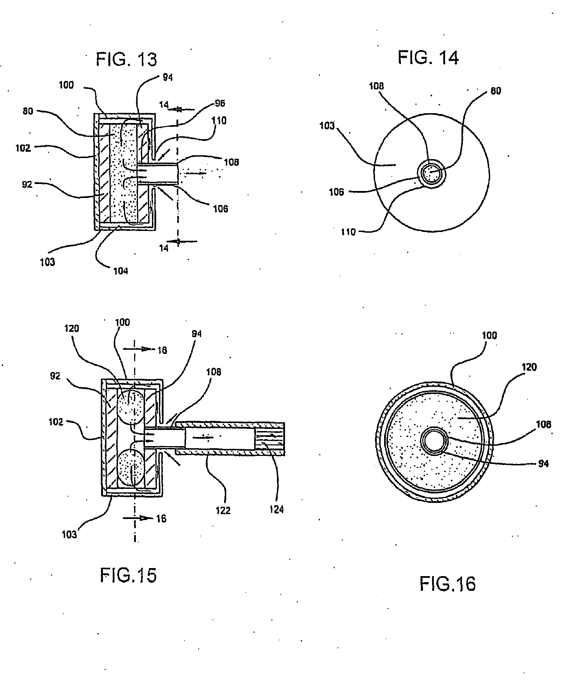

To enhance the heating efficiency of the embodiment of FIGs. 11 and 12, the assembly of a tobacco pillow 80, the disk heater 92, and the annular heater 94 may be enclosed in a housing 100 (see FIGs. 13 and 14). The housing 100 may include a wall 102 fabricated from an insulating material to reduce heat loss from the disk heater 92. Preferably the insulating wall 102 is substantially coextensive with the disk heater 92. The housing 100 also includes a baffle portion 103 attached to the insulating wall 102 and operable to direct airflow into the tobacco mass. The baffle 103 is spaced radially from both the disk heater 92 and the annular heater 94. Moreover, the baffle 103 is spaced axially in front of the annular heater 94. Although the actuation system is not schematically shown in FIG. 13, the actuation system for FIG. 13 is like that of FIG. 1 and includes conductors, batteries, and a switch.

With that arrangement, the baffle 103 forms an internal channel 104, which receives ambient air through an annular opening 106 and directs that ambient air radially outwardly through a preheating passage, defined between the annular heater 94 and the baffle, to a collector substantially surrounding the peripheral edge of the pillow 80. As the air passes the annular heater 94 in the channel 104, the air is heated thereby reducing energy loss or waste from the annular heater 94. The heated air then passes radially inwardly through the pillow 80 between the disk heater 92 and the annular heater 94 and leaves through the generally circular opening 96.

A generally cylindrical connector tube 108 with a generally circular cross section is fitted into the opening 96 and provides a passageway for heated air with entrained tobacco volatiles leaving the pillow 80 through the opening 96. In addition, the tube 108 cooperates with the central opening 110 of the baffle 103 to define an annular inlet opening 106. As air leaves the tube 108, it cools and the entrained tobacco volatiles form an aerosol.

Use of a generally toriodal tobacco mass is depicted in FIGs. 15 and 16. Here, the structural characteristics of the electric tobacco heater are essentially the same as those of the heater described above in connection with FIGs. 13 and 14. Although the actuation system is not schematically shown in FIG. 15, the actuation system for FIG. 15 is like that of FIG. 1 and includes conductors, batteries, and a switch. The principal difference being the tobacco mass, which is used. Here the tobacco mass 120 is generally toroidally shaped, i.e., shaped like a donut. The tobacco donut 120 is sandwiched between the disk heater 92 and the annular heater 94 so that the tobacco donut contacts both heaters. In this way, air is forced to travel through the tobacco donut 120. Preferably, the tobacco donut does not extend outwardly to touch the shell 103 so that the passageway for air is not obstructed.

The electric tobacco heater (FIG. 15) is shown with a mouthpiece 122 attached at one end to the tube 108. At the other end, the mouthpiece 122 may include a suitable filter 124.

It is also contemplated that the tobacco mass may be arranged so that successive portions of the tobacco mass can be advanced to the heating system. Individual tobacco mass portions may be used for a time corresponding generally to one puff, to multiple puffs, or to correspond to a time comparable to the smoking of a conventional cigarette. The embodiments of FIGs. 9-12 are particularly well suited for such applications. For example, successive tobacco mass portions may be carried on a tape and advanced into position relative to the heating system by a suitable indexing arrangement. Alternatively, the tobacco mass portions may be carried by a cassette, or may comprise discrete packages.

The terms and phases used herein are not to be interpreted with mathematical or geometric precision, rather geometric terminology is to be interpreted as meaning approximating or similar to the geometric terms and concepts. Where the term "about" is used in relation to a number, it is intended that such number has a tolerance of plus or minus 5%. Similarly, such terms as "generally" and "substantially are intended to encompass both precise meanings of the associated terms and concepts as well as to provide reasonable latitude which is consistent with form, function, and/or meaning.

It will now be apparent to those skilled in the art that this specification describes a new, useful, and nonobvious smokeless electric tobacco smoking system. It will also be apparent to those skilled in the art that numerous modifications, variations, substitutes, and equivalents exist for various aspects of the invention that have been described in the detailed description above. Accordingly, it is expressly intended that all such modifications, variations, substitutions, and equivalents that fall within the scope of the invention, as defined by the appended claims, be embraced thereby.

## Claims

1. A combustionless tobacco smoking system comprising:
a tobacco mass (24); and
heating apparatus, electrically operated, operable to heat the tobacco mass (24) to a temperature in the range of 150°C to 220°C, defining a heat transfer channels through which air is directed, **characterized in that** the heating apparatus comprises a heater rod (72) having an end extending from the tobacco mass (24), and a heater disk (70) attached to the heater rod.

2. The combustionless tobacco smoking system of Claim 1 wherein:
(a) the tobacco mass has a rotationally symmetric shape; and/or
(b) the tobacco. mass is enclosed in a carrier; and/or
(c) at least part of the surface of the tobacco mass conforms to the heating apparatus.

3. The combustionless tobacco smoking system of Claim 2, wherein:
(a) the tobacco mass has a generally cylindrical shape; and/or
(b) the tobacco mass has a disk shape; and/or
(c) the tobacco mass is generally toroidal.

4. The combustionless tobacco smoking system of Claim 3, wherein:
(a) the cylindrical shape is solid; and/or
(b) the cylindrical shape is hollow.

5. The combustionless tobacco smoking system of Claim 2, wherein:
(a) the carrier is paper, and/or
(b) the carrier is a mesh material.

6. The combustionless tobacco smoking system of Claim 1 further comprising a substantially cylindrical heating element and further including a shroud which receives the substantially cylindrical heating element.

7. The combustionless tobacco smoking system of Claim 6, wherein the substantially cylindrical heating element and the shroud define a preheating chamber for air.

8. The combustionless tobacco smoking system of Claim 1, further including a mouthpiece having an end in fluid communication with the tobacco mass.

9. The combustionless tobacco smoking system of Claim 8, wherein:
(a) the mouthpiece includes a second end having a filter; and/or
(b) the mouthpiece is attached between the tobacco mass and the heater.

10. The combustion less tobacco smoking system of Claim 1, wherein the heater disk includes air passages.

11. The combustionless tobacco smoking system of Claim 1 further including a baffle substantially surrounding the tobacco mass and operable to direct airflow into the tobacco mass.

## Patentansprüche

1. Verbrennungsfreies Tabakrauchsystem, das Folgendes umfasst:
eine Tabakmasse (24) und
eine elektrisch betriebene Heizvorrichtung zum Erhitzen der Tabakmasse (24) auf eine Temperatur im Bereich von 150°C bis 220°C, die einen Wärmetransferkanal definiert, durch den Luft gelenkt wird, **dadurch gekennzeichnet, dass** die Heizvorrichtung einen Heizstab (72) mit einem sich von der Tabakmasse (24) erstreckenden Ende und eine Heizscheibe (70) umfasst, die an dem Heizstab befestigt ist.

2. Verbrennungsfreies Tabakrauchsystem nach Anspruch 1, wobei:
(a) die Tabakmasse eine drehsymmetrische Gestalt hat; und/oder
(b) die Tabakmasse in einem Träger eingeschlossen ist; und/oder
(c) wenigstens ein Teil der Oberfläche der Tabakmasse mit der Heizvorrichtung konform ist.

3. Verbrennungsfreies Tabakrauchsystem nach Anspruch 2, wobei:
(a) die Tabakmasse eine allgemein zylindrische Gestalt hat; und/oder
(b) die Tabakmasse eine Scheibengestalt hat; und/oder
(c) die Tabakmasse allgemein ringförmig ist.

4. Verbrennungsfreies Tabakrauchsystem nach Anspruch 3, wobei:
(a) die zylindrische Gestalt massiv ist; und/oder
(b) die zylindrische Gestalt hohl ist.

5. Verbrennungsfreies Tabakrauchsystem nach Anspruch 2, wobei:
(a) der Träger Papier ist; und/oder
(b) der Träger ein Netzmaterial ist.

6. Verbrennungsfreies Tabakrauchsystem nach Anspruch 1, das ferner ein im Wesentlichen zylindrisches Heizelement umfasst und ferner eine Hülle beinhaltet, die das im Wesentlichen zylindrische Heizelement aufnimmt.

7. Verbrennungsfreies Tabakrauchsystem nach Anspruch 6, wobei das im Wesentlichen zylindrische Heizelement und die Hülle eine Vorheizkammer für Luft definieren.

8. Verbrennungsfreies Tabakrauchsystem nach Anspruch 1, das ferner ein Mundstück mit einem Ende in Fluidverbindung mit der Tabakmasse beinhaltet.

9. Verbrennungsfreies Tabakrauchsystem nach Anspruch 8, wobei:
(a) das Mundstück ein zweites Ende mit einem Filter beinhaltet; und/oder
(b) das Mundstück zwischen der Tabakmasse und der Heizvorrichtung angebracht ist.

10. Verbrennungsfreies Tabakrauchsystem nach Anspruch 1, wobei die Heizscheibe Luftpassagen beinhaltet.

11. Verbrennungsfreies Tabakrauchsystem nach Anspruch 1, das ferner eine Ablenkplatte beinhaltet, die die Tabakmasse im Wesentlichen umgibt und die Aufgabe hat, den Luftstrom in die Tabakmasse zu lenken.

## Revendications

1. Système à fumer du tabac sans combustion, comprenant :
une masse de tabac (24); et
un appareil chauffant actionné électriquement, opérationnel pour chauffer la masse de tabac (24) à une température dans la plage de 150°C à 220°C, définissant un canal de transfert thermique dont l'air est dirigé, **caractérisé en ce que** l'appareil chauffant comprend un tige chauffante (72) ayant une extrémité s'étendant de la masse de tabac (24) et un disque chauffant (70) attaché à la tige chauffante.

2. Le système à fumer du tabac sans combustion de la revendication 1, dans lequel :
(a) la masse de tabac a une forme à symétrie de révolution; et/ou
(b) la masse de tabac est renfermée dans un support; et/ou
(c) une partie au moins de la surface de la masse de tabac épouse la forme de l'appareil chauffant.

3. Le système à fumer du tabac sans combustion de la revendication 2, dans lequel :
(a) la masse de tabac a une forme généralement cylindrique; et/ou
(b) la masse de tabac a une forme de disque; et/ou
(c) la masse de tabac est généralement toroïdale.

4. Le système à fumer du tabac sans combustion de la revendication 3, dans lequel :
(a) la forme cylindrique est pleine; et/ou
(b) la forme cylindrique est creuse.

5. Le système à fumer du tabac sans combustion de la revendication 2, dans lequel :
(a) le support est du papier; et/ou
(b) le support est une matière maillée.

6. Le système à fumer du tabac sans combustion de la revendication 1, comprenant en outre un élément chauffant sensiblement cylindrique et incluant en plus une gaine qui reçoit l'élément chauffant sensiblement cylindrique.

7. Le système à fumer du tabac sans combustion de la revendication 6, dans lequel l'élément chauffant sensiblement cylindrique et la gaine définissent une chambre préchauffante pour l'air.

8. Le système à fumer du tabac sans combustion de la revendication 1, comprenant en outre une embouchure ayant une extrémité en communication fluide avec la masse de tabac.

9. Le système à fumer du tabac sans combustion de la revendication 8, dans lequel :
(a) l'embouchure comprend une deuxième extrémité dotée d'un filtre; et/ou
(b) l'embouchure est attachée entre la masse de tabac et l'appareil chauffant.

10. Le système à fumer du tabac sans combustion de la revendication 1, dans lequel le disque chauffant comprend des passages d'air.

11. Le système à fumer du tabac sans combustion de la revendication 1, comprenant en outre un déflecteur entourant sensiblement la masse de tabac et opérationnel pour diriger l'écoulement d'air dans la masse de tabac.
